# EUROPÄISCHE PATENTSCHRIFT

(11) **EP 0 954 266 B1**
(45) Veröffentlichungstag und Bekanntmachung des Hinweises auf die Patenterteilung: **26.04.2006**
(21) Anmeldenummer: 98905309.5
(22) Anmeldetag: 17.01.1998
(51) Int. Cl.: A61F 13/10, A61F 5/058, A61F 5/01

(54) **SELBSTKLEBENDE FERTIGBANDAGE ZUR STRECKUNGS- ODER BEUGUNGSEINSCHRÄNKUNG DES FINGERGRUNDGELENKS UND DES HANDGELENKS**
SELF-ADHESIVE READY-MADE BANDAGE FOR RESTRICTING STRETCHING OR BENDING OF THE METACARPOPHALANGEAL JOINT AND OF THE WRIST
BANDAGE PREFORME AUTO-ADHESIF POUR LIMITER L'EXTENSION OU LA FLEXION DE L'ARTICULATION METACARPOPHALANGIENNE DU DOIGT ET CELLE DU POIGNET

(30) Priorität: 23.01.1997 DE 19702301; 23.01.1997 DE 19702302
(43) Veröffentlichungstag der Anmeldung: 10.11.1999
(73) Patentinhaber: Beiersdorf Aktiengesellschaft, 20245 Hamburg (DE)
(72) Erfinder: HIMMELSBACH, Peter, D-21614 Buxtehude (DE)
(86) Internationale Anmeldenummer: PCT/EP1998/000251
(87) Internationale Veröffentlichungsnummer: WO 1998/032405

(56) Entgegenhaltungen:
- EP-A- 0 039 323
- DE-C- 734 888
- FR-A- 752 041
- US-A- 2 563 689
- US-A- 2 875 758
- US-A- 3 880 159

## Beschreibung

Die Erfindung betrifft eine einseitig selbstklebend beschichtete Fertigbandage zur Strekkungs- oder Beugungseinschränkung des Fingergrundgelenks.

Die funktionelle Verbandtechnik, das sogenannte Taping, ist eine Behandlungsmethode zur Prophylaxe und Therapie von Verletzungen, Krankheiten und Veränderungen am Bewegungsapparat. Taping hat zum Ziel, die Kapsel-Band-Strukturen gezielt nachzubilden, und dadurch eine selektive Unterstützung und Stabilisierung zu erreichen.

Der eigentliche Tapeverband wird dabei streifenweise aus vorzugsweise unelastischen selbstklebenden Bändern, sogenannten Zügeln, oder in Verbindung mit kurzzugelastischen selbstklebenden Bändern angelegt. Er schützt, stützt und entlastet gefährdete, geschädigte oder gestörte Anteile einer Funktionseinheit. Er erlaubt die selektive Belastung im schmerzfreien Bewegungsraum, verhindert aber extreme oder schmerzhafte Bewegungen.

Das Anlegen derartiger Verbände erfordert jedoch fachmännisches Können und Erfahrung und kann deshalb in aller Regel nicht von Laien ohne Taping-Erfahrung ausgeführt werden.

Für das Fingergrundgelenk, das insbesondere bei sportlicher Betätigung oft sehr hohem mechanischen Streß ausgesetzt ist, der zu Distorsionen und Kontusionen aber auch zu Überlastungsreizungen an der Kapsel führen kann, verlangt der Fachmann aber eine einfach aufgebaute und gleichzeitig problemlos anzulegende Fertigbandage, die besonders bei leichteren Verletzungen einen positiven Einfluß auf den Heilungsprozeß nimmt.

Ähnliches trifft auch auf das Handgelenk zu, so daß die gleichen Anforderungen wie beim Fingergrundgelenk an die Fertigbandage gestellt werden.

Eine derartige Fertigbandage sollte aber auch der Laie anlegen können, so daß diesem eine preiswerte und wenig zeitintensive Hilfestellung durch die Fertigbandage geboten würde.

FR-A-230 75 18 beschreibt eine Festigbandage, die für jedes Gelenk des menschlichen Körpers anwandbar sein soll, explizit jedoch nur für Spring-, Ellenbogen- und Handgelenk.

Im Einzelnen offenbart die Druckschrift eine selbst klebend beschichtete Fertigbandage zur Unterstützung und Beugungseinschrinkung des menschlichen Gelenks, wobei an den Enden eines länglichen Streifens zwei Befestigungszügel und im mittleren Bereich zwei weitere Befestigungflügel angebracht sind.

Die Bandage ist im Längsrichtung nicht dehnbar, jedoch in Querrichtung elastisch.

Aufgabe der Erfindung war es deshalb, eine Fertigbandage zur Verfügung zu stellen, die aufgrund ihrer Ausgestaltung, ihres Materials und ihrer Eigenschaften zur Unterstützung des Heilungsprozesses bei Verletzungen im Fingergrundgelenk durch Einschränkung der Streckungs- oder Beugungsfähigkeit geeignet ist und die darüber hinaus vom Anwender in einfacher Weise angelegt werden kann.

Gelöst wird diese Aufgabe durch eine Fertigbandage gemäß Anspruch 1.

Demnach besteht die auf mindestens einer Seite selbstklebend beschichtete Fertigbandage zur Streckungs- oder Beugungseinschränkung des Fingergrundgelenks mit einer Höchstzugkraft von nicht weniger als 50 N/cm und einer Höchstzugkraftdehnung von weniger als 20%, aus
a.) einem länglichen Streifen (1), der 12 cm bis 30 cm lang ist und sich in Richtung des Kopfbereiches (13) verjüngt,
b.) mindestens einem Kurzzügel (41, 42), der zumindest an einer der beiden Längsseiten im Kopfbereich (13) des Streifens (1) angebracht ist,
c.) ein oder zwei Zügeln (21, 22), die im Fußbereich (11, 12) des Streifens (1) an den Längsseiten angebracht sind,
d.) wobei die Zügel (21, 22, 41, 42) mit dem länglichen Streifen (1) jeweils einen Winkel zwischen 30° bis 150° einschließen und
wobei die Fertigbandage an dem jeweiligen Finger anlegbar ist.

Vorteilhafterweise sind der erste Zügel und/oder der Kurzzügel in einem Winkel von 90° zum länglichen Streifen angebracht. Dies gilt auch für den Fall, daß statt eines ersten Zügels zwei oder mehrere erste Zügel beziehungsweise statt eines Kurzzügels zwei oder mehrere Kurzzügel vorgesehen sind.

In einer ersten bevorzugten Ausführungsform der erfindungsgemäßen Fertigbandage, die zur Beugungseinschränkung des Fingergrundgelenks dient und bei der an dem länglichen Streifen auf jeder der beiden Längsseiten jeweils ein erster Zügel und ein Kurzzügel angeformt sind, sind der längliche Streifen etwa 12 cm bis 30 cm lang und an der Querseite im Fußbereich 3 cm bis 7 cm beziehungsweise an der Querseite im Kopfbereich 1 cm bis 3 cm breit, die ersten Zügel jeweils etwa 5 cm bis 30 cm lang und 2 cm bis 6 cm breit und die Kurzzügel jeweils etwa 3 cm bis 6 cm lang und 1 bis 3 cm breit.
Die Abmessungen der Fertigbandage richten sich natürlich nach der Größe der Hand, an der die Fertigbandage angelegt wird. Für eine durchschnittliche Hand eines Erwachsenen weisen die einzelnen Teile der Fertigbandage die folgenden Maße auf:
- Der längliche Streifen ist etwa 20 cm lang, an der Querseite im Fußbereich 6 cm und an der Querseite im Kopfbereich 2 cm breit.
- Die ersten Zügel sind 22 cm lang und 5 cm breit.
- Die Kurzzügel sind 5 cm lang und 2 cm breit.

Alle Streifen und Zügel können darüber hinaus auch abgerundete Ecken aufweisen, um die Gefahr des ungewollten Ablösens der verklebten Fertigbandage zu verringern.

In dem mittleren Bereich des länglichen Streifens kann ebenfalls zumindest an einer der beiden Längsseiten ein zweiter Zügel angebracht sein, der mit dem länglichen Streifen einen Winkel zwischen 30° bis 150° einschließt und der auf der palmaren Fläche der Hand angelegt wird.

Vorteilhafterweise sind der erste Zügel, der zweite Zügel und/oder der Kurzzügel in einem Winkel von 90° zum länglichen Streifen angebracht. Dies gilt auch für den Fall, daß statt eines ersten oder zweiten Zügels jeweils zwei oder mehrere Zügel beziehungsweise statt eines Kurzzügels zwei Kurzzügel vorgesehen sind.

Weiterhin kann sich der längliche Streifen beginnend im mittleren Bereich in Richtung des Kopfbereiches verjüngen.

In einer zweiten bevorzugten Ausführungsform der erfindungsgemäßen Fertigbandage, die zur Streckungseinschränkung des Fingergrundgelenks dient und bei der an dem länglichen Streifen auf jeder der beiden Längsseiten jeweils ein erster Zügel, ein zweiter Zügel und ein Kurzzügel angeformt sind, sind der längliche Streifen etwa 12 cm bis 30 cm lang und an der Querseite im Fußbereich 3 cm bis 6 cm beziehungsweise an der Querseite im Kopfbereich 1 cm bis 3 cm breit, wobei die Verjüngung des länglichen Streifens gemessen von der Querseite im Fußbereich nach etwa 6 cm bis 22 cm einsetzt, die ersten Zügel jeweils etwa 5 cm bis 30 cm lang und 2 cm bis 6 cm breit, die zweiten Zügel jeweils etwa 5 bis 30 cm lang und 2 cm bis 6 cm breit und die Kurzzügel jeweils etwa 3 cm bis 6 cm lang und 1 cm bis 3 cm breit.
Die Abmessungen der Fertigbandage richten sich natürlich nach der Größe der Hand, an der die Fertigbandage angelegt wird. Für eine durchschnittliche Hand eines Erwachsenen weisen die einzelnen Teile der Fertigbandage die folgenden Maße auf:
- Der längliche Streifen ist etwa 18 cm lang, an der Querseite im Fußbereich 6 cm und an der Querseite im Kopfbereich 2 cm breit, wobei die Verjüngung des länglichen Streifens gemessen von der Querseite im Fußbereich nach 12 cm einsetzt.
- Die ersten Zügel sind 22 cm lang und 5 cm breit.
- Die zweiten Zügel sind 22 cm lang und 3,75 cm breit.
- Die Kurzzügel sind 4 cm lang und 2 cm breit.

Alle Streifen und Zügel können darüber hinaus auch abgerundete Ecken aufweisen, um die Gefahr des ungewollten Ablösens der verklebten Fertigbandage zu verringern.

Als besonders vorteilhaft hat sich erwiesen, daß an den Stellen, an denen der längliche Streifen, die ersten Zügel, die zweiten Zügel und/oder die Kurzzügel zusammentreffen, Ausnehmungen oder Aussparungen vorhanden sind. Diese Ausnehmungen verhindern das Einreißen der Fertigbandage an den Punkten, die die höchste Beanspruchung insbesondere beim Anlegen aufweisen. Auf der anderen Seite erhöhen die Aussparungen die Flexibilität der Fertigbandage, so daß auf der anderen Seite das Anlegen erleichtert wird.

Weiterhin vorzugsweise besteht die erfindungsgemäße selbstklebende Fertigbandage aus einem unelastischen Gewebe oder Gewirke. Fallweise können sich auch elastische oder plastische Anteile in Längs- oder in Querrichtung des Trägermaterials vorteilhaft auf die Anwenderempfindung auswirken. Weiterhin können auch Vliesstoffe oder Schäume oder Papier eingesetzt werden, wenn diese eine ausreichende Festigkeit aufweisen.

Vorzugsweise kann das Trägermaterial aus Baumwolle bestehen.

Die Fertigbandage ist auf der Seite, die auf der Haut aufgelegt wird, mit einer der bekannten gut haftenden Selbstklebemassen auf Basis von Kautschuk (vorzugsweise einer Zink-Kautschuk-Masse) oder synthetischen Polymeren beschichtet.
Bei der Selbstklebemasse kann es sich um Lösemittel-, Dispersions/Emulsionssysteme handeln, aber auch 100%- Selbstklebemassensysteme können Anwendung finden. Vorteilhaft weisen die Massen weitere Eigenschaften wie gute Hautverträglichkeit oder Luft- und Wasserdampfdurchlässigkeit auf.
Die Selbstklebemasse kann zur Erzielung der genannten Effekte mit ungefähr 120 g/m² partiell aufgetragen sein, so zum Beispiel unter Verwendung des Thermosiebdrucks mit einer 14 Mesh-Siebschablone mit einer Siebstärke von 300 µm.

Die Klebeschicht ist bis zum Gebrauch der Bandage mit einem klebstoffabweisend ausgerüstetem Blattmaterial wie beispielsweise silikonisiertem Papier, das darüber hinaus zur besseren Anwendung perforiert werden kann, oder einer Folie aus Kunststoff abgedeckt.
Das Material kann dabei in mehrere Abschnitte aufgegliedert sein, um das Anlegen der Fertigbandage durch sukzessives Ablösen der einzelnen Abschnitte zu erleichtem.

Die Fertigbandage ist universell zur Streckungs- oder Beugungseinschränkung des Fingergrundgelenks einsetzbar.

Der Zuschnitt der Fertigbandage ist davon abhängig, an welcher Hand die Fertigbandage angelegt werden soll. Dementsprechend gibt es zwei spiegelsymmetrische Ausführungsformen der erfindungsgemäßen Fertigbandage.

Anhand der im folgenden beschriebenen Figuren sollen eine besonders vorteilhafte Ausführungsform der erfindungsgemäßen Fertigbandage sowie deren Anwendung näher dargestellt werden.

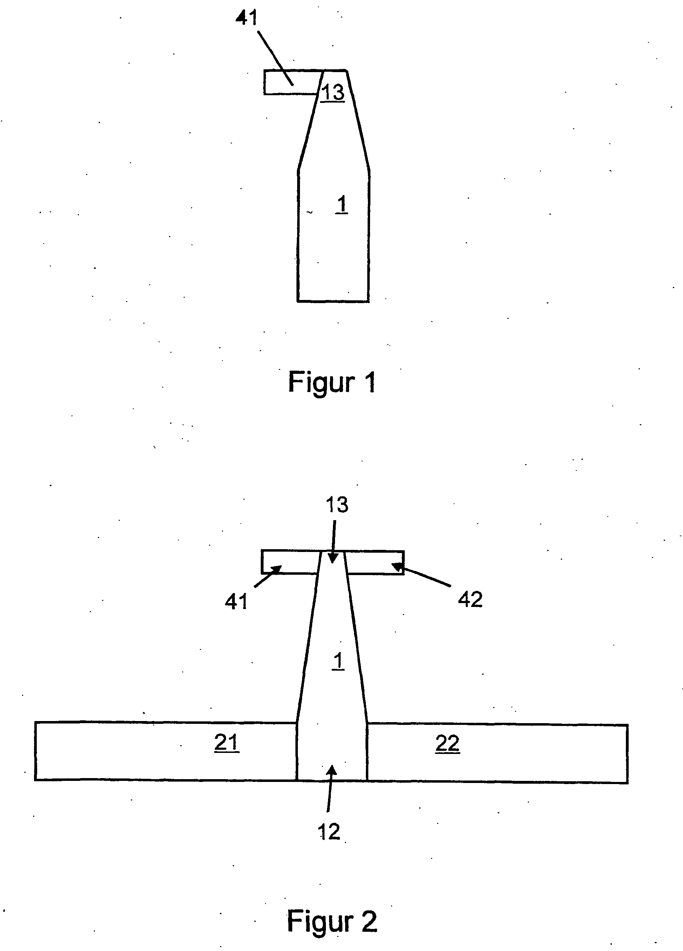

Die Figur 1 zeigt eine nicht erfindungsgemäße Fertigbandage in einer ihrer vereinfachten Ausführungsform. Die Fertigbandage (1) setzt sich aus mehreren Abschnitten zusammen. Der zentrale Abschnitt wird von einem länglichen Streifen (1) gebildet, der sich in Richtung des Kopfbereiches (13) verjüngt.
Im Kopfbereich (13) des länglichen Streifen (1) ist ein Kurzzügel (41) vorgesehen, der eine rechtwinklige Form aufweist und in einem Winkel von 90° zur Orientierung des länglichen Streifens (1) angebracht ist.

Zum Anlegen der Fertigbandage wird als erstes jeweils das klebstoffabweisende Material von dem zu verklebenden Abschnitt der Fertigbandage entfemt.

Zunächst kann der längliche Streifen (1) auf der palmaren Fläche der Hand flächig angelegt, so daß dieser ausgehend vom hier betroffenen Mittelfinger über das Handgelenk bis zum Unterarm verläuft. Dabei sollte das Handgelenk eine Flexion von mindestens 30° aufweisen. Der Kurzzügel (31) wird zirkulär fest um den Mittelfinger gewickelt, wobei mindestens ein Teil, bevorzugt der gesamte Finger umschlossen ist. Vorteilhaft ist ein ausreichendes Anmodellieren des Fertigverbandes.

Die Figur 2 zeigt die Fertigbandage in der ersten bevorzugten Ausführungsform. Der zentrale Abschnitt wird von einem länglichen Streifen (1) gebildet, der sich in Richtung des Kopfbereiches (13) verjüngt. Im Fußbereich (12) des länglichen Streifens (1) sind auf beiden Längsseiten jeweils ein Zügel (21, 22) im rechten Winkel zum länglichen Streifen (1) angeformt, die eine im wesentlichen rechteckige Form aufweisen. Weiterhin sind im Kopfbereich (13) des länglichen Streifen (1) zwei Kurzzügel (41, 42) vorgesehen, die ebenfalls eine rechtwinklige Form aufweisen und in einem Winkel von 90° zum länglichen Streifen (1) angebracht sind, die aber eine kleinere Grundfläche besitzen als die Zügel (21, 22).

Zum Anlegen der Fertigbandage wird als erstes jeweils das klebstoffabweisende Material von dem zu verklebenden Abschnitt der Fertigbandage entfernt.
Zunächst kann der längliche Streifen (1) auf der dorsalen Fläche der Hand angelegt, so daß dieser ausgehend vom hier betroffenen Mittelfinger über das Handgelenk bis zum Unterarm verläuft. Dabei sollte das Handgelenk eine Flexion von mindestens 30° aufweisen. Die Kurzzügel (41, 42) werden distal des Fingergrundgelenks fest um den Mittelfinger gewickelt, wobei mindestens ein Teil, bevorzugt der gesamte Finger umschlossen ist.
Die Zügel (21, 22) werden proximal des Handgelenks zirkulär um den Unterarm geführt und fixiert.

Der sorgfältig angelegte Fertigbandage ist in der Lage, die Beugungsfähigkeit des Mittelfingers einzuschränken, wobei ein zusätzlich angelegter Ankerstreifen um den Unterarm die Wirkung der Fertigbandage noch verstärken kann.

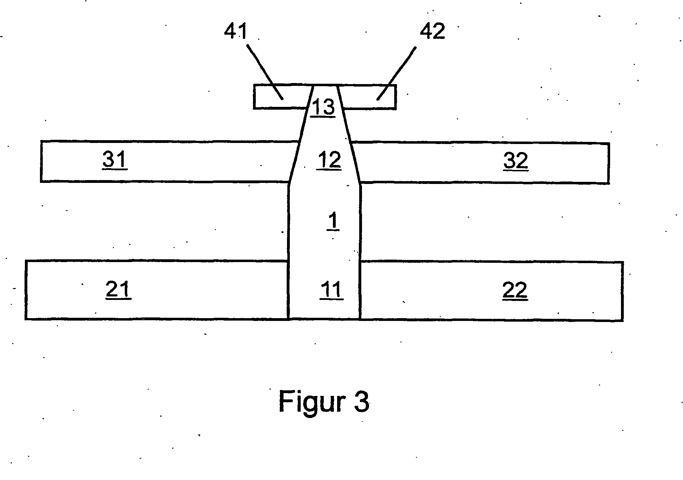

Die Figur 3 zeigt die Fertigbandage in der zweiten bevorzugten Ausführungsform. Der zentrale Abschnitt wird von einem länglichen Streifen (1) gebildet. Im Fußbereich (11) des länglichen Streifens (1) sind auf beiden Längsseiten jeweils ein erster Zügel (21, 22) im rechten Winkel zum länglichen Streifen (1) angeformt, die eine im wesentlichen rechteckige Form aufweisen. Der längliche Streifen (1) verjüngt sich beginnend im mittleren Bereich (12) in Richtung des Kopfbereiches (13). Im mittleren Bereich (12) sind weiterhin auf beiden Längsseiten jeweils ein zweiter Zügel (31, 32) in einem Winkel von 90° zum länglichen Streifen (1) vorhanden. Die zweiten Zügel (31, 32) sind auch im wesentlichen rechteckig geformt, besitzen aber geringe Abmessungen als die ersten Zügel (21, 22).
Weiterhin sind im Kopfbereich (13) des länglichen Streifen (1) zwei Kurzzügel (41, 42) vorgesehen, die ebenfalls eine rechtwinklige Form aufweisen und in einem Winkel von 90° zum länglichen Streifen (1) angebracht sind, die aber eine kleinere Grundfläche aufweisen als die zweiten Zügel (31, 32).

Zum Anlegen der Fertigbandage wird als erstes jeweils das klebstoffabweisende Material von dem zu verklebenden Abschnitt der Fertigbandage entfernt.

Zunächst kann der längliche Streifen (1) auf der palmaren Fläche der Hand angelegt werden, so daß dieser ausgehend vom hier betroffenen Mittelfinger über das Handgelenk bis zum Unterarm verläuft. Dabei sollte das Handgelenk eine Flexion von mindestens 30° aufweisen.
Die Kurzzügel (41, 42) werden distal des Fingergrundgelenks fest um den Mittelfinger gewickelt, wobei mindestens ein Teil, bevorzugt der gesamte Finger umschlossen ist.

Die ersten Zügel (21, 22) werden proximal des Handgelenks zirkulär um den Unterarm geführt und fixiert.
Die zweiten Zügel (31, 32) werden ausgehend von der palmaren Fläche der Hand bis zum Handrücken geführt und dabei stets auf den entsprechenden Hautpartien fest angedrückt.

Der sorgfältig angelegte Fertigbandage ist in der Lage, die Streckungsfähigkeit des Mittelfingers einzuschränken, wobei ein zusätzlich angelegter Ankerstreifen um den Unterarm die Wirkung der Fertigbandage noch verstärken kann.

## Patentansprüche

1. Mindestens auf einer Seite selbstklebend beschichtete Fertigbandage zur Streckungs- oder Beugungseinschränkung des Fingergrundgelenks mit einer Höchstzugkraft von nicht weniger als 50 N/cm und einer Höchstzugkraftdehnung von weniger als 20%, umfassend
a.) einen länglichen Streifen (1), der 12 cm bis 30 cm lang ist und sich in Richtung des Kopfbereiches (13) verjüngt,
b.) mindestens einen Kurzzügel (41, 42), der zumindest an einer der beiden Längsseiten im Kopfbereich (13) des Streifens (1) angebracht ist,
c.) ein oder zwei Zügel (21, 22), die im Fußbereich (11, 12) des Streifens (1) an den Längsseiten angebracht sind,
d.) wobei die Zügel (21, 22, 41, 42) mit dem länglichen Streifen (1) jeweils einen Winkel zwischen 30° bis 150° einschließen und
wobei die Fertigbandage an dem jeweiligen Finger anlegbar ist.

2. Selbstklebende Fertigbandage gemäß Anspruch 1, **dadurch gekennzeichnet, dass** an beiden Längsseiten des länglichen Streifen (1) jeweils zwei erste Zügel (21, 22) und zwei Kurzzügel (41, 42) angeformt sind.

3. Selbstklebende Fertigbandage gemäß Anspruch 2, **dadurch gekennzeichnet, dass**
der längliche Streifen (1) an der Querseite im Fußbereich (11,12) 3 cm bis 7 cm, insbesondere 6 cm, beziehungsweise an der Querseite im Kopfbereich (13) 1 cm bis 3 cm, insbesondere 2 cm, breit ist,
die ersten Zügel (21, 22) etwa 5 cm bis 30 cm, insbesondere 22 cm, lang und 2 cm bis 6 cm, insbesondere 5 cm, breit sind,
die Kurzzügel (41, 42) etwa 3 cm bis 6 cm, insbesondere 5 cm, lang und 1 cm bis 3 cm, insbesondere 2 cm, breit sind.

4. Selbstklebende Fertigbandage gemäß Anspruch 3, **dadurch gekennzeichnet, dass** der längliche Streifen (1) 20 cm lang ist.

5. Selbstklebende Fertigbandage nach einem der Ansprüche 1 bis 4, **dadurch gekennzeichnet, dass** im mittleren Bereich (12) des Streifens (1) ein oder zwei Zügel (31, 32) an den Längsseiten angebracht sind, wobei die Zügel (31, 32) mit dem länglichen Streifen (1) jeweils einen Winkel zwischen 30° bis 150° einschließen und auf der palmaren Fläche der Hand anlegbar sind.

6. Selbstklebende Fertigbandage gemäß einem der Ansprüche 1 bis 5, **dadurch gekennzeichnet, dass** an beiden Längsseiten des länglichen Streifen (1) jeweils zwei erste Zügel (21, 22), zwei zweite Zügel (31, 32) und zwei Kurzzügel (41, 42) angeformt sind.

7. Selbstklebende Fertigbandage gemäß Anspruch 6, **dadurch gekennzeichnet, dass** der längliche Streifen (1) an der Querseite im Fußbereich (11) 3 cm bis 7 cm, insbesondere 6 cm, beziehungsweise an der Querseite im Kopfbereich (13) 1 cm bis 3 cm, insbesondere 2 cm, breit ist,
wobei die Verjüngung des länglichen Streifens (1) gemessen von der Querseite im Fußbereich (11) nach etwa 6 cm bis 22 cm, insbesondere 12 cm, einsetzt, die ersten Zügel (21, 22) etwa 5 cm bis 30 cm, insbesondere 22 cm, lang und 2 cm bis 6 cm, insbesondere 5 cm, breit sind,
die zweiten Zügel (31, 32) etwa 5 cm bis 30 cm, insbesondere 22 cm, lang und 2 bis 6 cm, insbesondere 3,75 cm, breit sind,
die Kurzzügel (41, 42) etwa 3 cm bis 6 cm, insbesondere 4 cm, lang und 1 cm bis 3 cm, insbesondere 2 cm, breit sind.

8. Selbstklebende Fertigbandage gemäß Anspruch 7, **dadurch gekennzeichnet, dass** der längliche Streifen (1) 18 cm lang ist.

9. Selbstklebende Fertigbandage nach einem der Ansprüche 1 bis 8, **dadurch gekennzeichnet, dass** die Zügel (21, 22, 31, 32) und/oder Kurzzügel (41, 42) jeweils in einem Winkel von 90° zum länglichen Streifen (1) angebracht sind.

10. Selbstklebende Fertigbandage gemäß einem der Ansprüche 1 bis 9, **dadurch gekennzeichnet, dass** an den Stellen, an denen der längliche Streifen (1), die ersten Zügel (21, 22), die zweiten Zügel (31, 32) und/oder die Kurzzügel (41, 42) zusammentreffen, Ausnehmungen vorhanden sind.

11. Selbstklebende Fertigbandage gemäß einem der Ansprüche 1 bis 10, **dadurch gekennzeichnet, dass** die Fertigbandage aus einem unelastischem Trägermaterial besteht, insbesondere Vliese, Papiere, Schäume, Gewebe oder Gewirke.

12. Selbstklebende Fertigbandage gemäß Anspruch 11, **dadurch gekennzeichnet, dass** das Gewebe aus Baumwolle besteht.

13. Selbstklebende Fertigbandage gemäß einem der Ansprüche 1 bis 12, **dadurch gekennzeichnet, dass** die Fertigbandage auf ihrer selbstklebenden Seite mit klebstoffabweisendem Material abgedeckt ist.

## Claims

1. Ready-made bandage with a self-adhesive coating on at least one side, for restricting stretching or bending of the metacarpophalangeal joint, with a maximum tensile force of not less than 50 N/cm and a maximum tensile force extension of less than 20%, comprising
a) an oblong strip (1) which is 12 cm to 30 cm long and narrows in the direction towards the head area (13),
b) at least one short rein (41, 42) arranged on at least one of the two longitudinal sides in the head area (13) of the strip (1),
c) one or two reins (21, 22) arranged on the longitudinal sides in the bottom area (11, 12) of the strip (1),
d) the reins (21, 22, 41, 42) each enclosing an angle of between 30° and 150° with the oblong strip (1), and
said ready-made bandage being able to be applied on the finger in question.

2. Self-adhesive ready-made bandage according to Claim 1, **characterized in that** two first reins (21, 22) and two short reins (41, 42) are in each case formed integrally on both longitudinal sides of the oblong strip (1).

3. Self-adhesive ready-made bandage according to Claim 2, **characterized in that** the oblong strip (1) is 3 cm to 7 cm wide, in particular 6 cm wide, at the transverse side in the bottom area (11, 12), and is 1 cm to 3 cm wide, in particular 2 cm wide, at the transverse side in the head area (13),
the first reins (21, 22) are about 5 cm to 30 cm long, in particular 22 cm long, and are 2 cm to 6 cm wide, in particular 5 cm wide, and
the short reins (41, 42) are about 3 cm to 6 cm long, in particular 5 cm long, and are 1 cm to 3 cm wide, in particular 2 cm wide.

4. Self-adhesive ready-made bandage according to Claim 3, **characterized in that** the oblong strip (1) is 20 cm long.

5. Self-adhesive ready-made bandage according to one of Claims 1 to 4, **characterized in that** one or two reins (31, 32) are arranged on the longitudinal sides in the middle area (12) of the strip (1), which reins (31, 32) each enclose an angle of between 30° and 150° with the oblong strip (1) and can be applied on the palmar surface of the hand.

6. Self-adhesive ready-made bandage according to one of Claims 1 to 5, **characterized in that** two first reins (21, 22), two second reins (31, 32) and two short reins (41, 42) are in each case formed integrally on both longitudinal sides of the oblong strip (1).

7. Self-adhesive ready-made bandage according to Claim 6, **characterized in that** the oblong strip (1) is 3 cm to 7 cm wide, in particular 6 cm wide, at the transverse side in the bottom area (11), and is 1 cm to 3 cm wide, in particular 2 cm wide, at the transverse side in the head area (13), the narrowing of the oblong strip, measured from the transverse side in the bottom area (11), starting after about 6 cm to 22 cm, in particular after 12 cm, the first reins (21, 22) being about 5 cm to 30 cm long, in particular 22 cm long, and being 2 cm to 6 cm wide, in particular 5 cm wide, the second reins (31, 32) being about 5 cm to 30 cm long, in particular 22 cm long, and being 2 to 6 cm wide, in particular 3.75 cm wide, the short reins (41, 42) being about 3 cm to 6 cm long, in particular 4 cm long, and being 1 cm to 3 cm wide, in particular 2 cm wide.

8. Self-adhesive ready-made bandage according to Claim 7, **characterized in that** the oblong strip (1) is 18 cm long.

9. Self-adhesive ready-made bandage according to one of Claims 1 to 8, **characterized in that** the reins (21, 22, 31, 32) and/or short reins (41, 42) are each arranged at an angle of 90° to the oblong strip (1).

10. Self-adhesive ready-made bandage according to one of Claims 1 to 9, **characterized in that** cuttings are provided at the points where the oblong strip (1), the first reins (21, 22), the second reins (31, 32) and/or the short reins (41, 42) meet.

11. Self-adhesive ready-made bandage according to one of Claims 1 to 10, **characterized in that** the ready-made bandage is made of a non-elastic support material, in particular nonwovens, papers, foams, woven fabrics or knitted fabrics.

12. Self-adhesive ready-made bandage according to Claim 11, **characterized in that** the woven fabric is made of cotton.

13. Self-adhesive ready-made bandage according to one of Claims 1 to 12, **characterized in that** the ready-made bandage is covered, on its self-adhesive side, with anti-adhesive material.

## Revendications

1. Bandage préformé muni au moins d'un côté d'un revêtement auto-adhésif pour limiter l'extension ou la flexion de l'articulation métacarpophalangienne ayant une force d'étirement maximale d'au moins 50 N/cm et une élongation en traction maximale de moins de 20%, comprenant
a) un ruban allongé (1) mesurant de 12 cm à 30 cm de long et se rétrécissant dans la direction allant vers la région de la tête (13),
b) au moins une courte bride (41, 42) qui est prévue au moins sur l'un des deux côtés longitudinaux dans la région de la tête (13) du ruban (1),
c) une ou deux brides (21, 22) qui sont prévues dans la région de la base (11, 12) du ruban (1) sur les côtés longitudinaux,
d) les brides (21, 22, 41, 42) formant respectivement un angle compris entre 30° et 150° avec le ruban allongé (1) et
le bandage préformé pouvant être appliqué sur le doigt particulier.

2. Bandage préformé auto-adhésif selon la revendication 1, **caractérisé en ce que** deux premières brides (21, 22) et deux courtes brides (41, 42) sont respectivement formées sur les deux côtés longitudinaux du ruban allongé (1).

3. Bandage préformé auto-adhésif selon la revendication 2, **caractérisé en ce que**
le ruban allongé (1) mesure de 3 cm à 7 cm, notamment 6 cm de large, au niveau du côté transversal dans la région de la base (11, 12), respectivement de 1 cm à 3 cm, notamment 2 cm de large, au niveau du côté transversal dans la région de la tête (13),
les premières brides (21, 22) mesurent approximativement 5 cm à 30 cm, notamment 22 cm de long et 2 cm à 6 cm, notamment 5 cm de large,
les courtes brides (41, 42) mesurent approximativement 3 cm à 6 cm, notamment 5 cm de long et 1 cm à 3 cm, notamment 2 cm de large.

4. Bandage préformé auto-adhésif selon la revendication 3, **caractérisé en ce que** le ruban allongé (1) mesure 20 cm de long.

5. Bandage préformé auto-adhésif selon l'une quelconque des revendications 1 à 4, **caractérisé en ce qu'**une ou deux brides (31, 32) sont prévues sur les côtés longitudinaux dans la région centrale (12) du ruban (1), les brides (31, 32) formant respectivement un angle compris entre 30° et 150° avec le ruban allongé (1) et pouvant être appliquées sur la face palmaire de la main.

6. Bandage préformé auto-adhésif selon l'une quelconque des revendications 1 à 5, **caractérisé en ce que** deux premières brides (21, 22), deux deuxièmes brides (31, 32) et deux courtes brides (41, 42) sont respectivement formées sur les deux côtés longitudinaux du ruban allongé (1).

7. Bandage préformé auto-adhésif selon la revendication 6, **caractérisé en ce que**
le ruban allongé (1) mesure de 3 cm à 7 cm, notamment 6 cm de large, au niveau du côté transversal dans la région de la base (11), respectivement de 1 cm à 3 cm, notamment 2 cm de large, au niveau du côté transversal dans la région de la tête (13),
le rétrécissement du ruban allongé (1), mesuré depuis le côté transversal dans la région de la base (11), commence à environ 6 cm à 22 cm, notamment à 12 cm,
les premières brides (21, 22) mesurent approximativement 5 cm à 30 cm, notamment 22 cm de long et 2 cm à 6 cm, notamment 5 cm de large,
les deuxièmes brides (31, 32) mesurent approximativement 5 cm à 30 cm, notamment 22 cm de long, et de 2 à 6 cm, notamment 3,75 cm de large,
les courtes brides (41, 42) mesurent approximativement 3 cm à 6 cm, notamment 4 cm de long et 1 cm à 3 cm, notamment 2 cm de large.

8. Bandage préformé auto-adhésif selon la revendication 7, **caractérisé en ce que** le ruban allongé (1) mesure 18 cm de long.

9. Bandage préformé auto-adhésif selon l'une quelconque des revendications 1 à 8, **caractérisé en ce que** les brides (21, 22, 31, 32) et/ou les courtes brides (41, 42) sont prévues respectivement à un angle de 90° par rapport au ruban allongé (1).

10. Bandage préformé auto-adhésif selon l'une quelconque des revendications 1 à 9, **caractérisé en ce que** des évidements sont prévus aux endroits auxquels le ruban allongé (1), les premières brides (21, 22), les deuxièmes brides (31, 32) et/ou les courtes brides (41, 42) se rejoignent.

11. Bandage préformé auto-adhésif selon l'une quelconque des revendications 1 à 10, **caractérisé en ce que** le bandage préformé se compose d'un matériau de support non élastique, notamment des non-tissés, des papiers, des mousses, des tissus ou des tissus à mailles.

12. Bandage préformé auto-adhésif selon la revendication 11, **caractérisé en ce que** le tissu se compose de coton.

13. Bandage préformé auto-adhésif selon l'une quelconque des revendications 1 à 12, **caractérisé en ce que** le bandage préformé est recouvert sur son côté auto-adhésif d'un matériau anti-adhésif.
